(19) Europäisches Patentamt
European Patent Office
Office européen des brevets

(11) Publication number: 0 237 166
A2

(12) # EUROPEAN PATENT APPLICATION

(21) Application number: 87300855.1

(22) Date of filing: 30.01.87

(51) Int. Cl.³: C 07 D 311/64
C 07 K 15/02, A 61 K 31/35
A 61 K 37/02

(30) Priority: 31.01.86 US 825138

(43) Date of publication of application:
16.09.87 Bulletin 87/38

(84) Designated Contracting States:
AT BE CH DE ES FR GB GR IT LI LU NL SE

(71) Applicant: THE STATE OF OREGON ACTING BY AND THROUGH THE OREGON STATEBOARD OF HIGHER EDUCATION ON BEHALF OF OREGON STATE UNIVERSITY
P.O. Box 3175
Eugene Oregon 97403(US)

(72) Inventor: Gerwick, William H.
247 Peterson Road
Philomath, Oregon 97370(US)

(74) Representative: Brown, John David et al,
FORRESTER & BOEHMERT Widenmayerstrasse 4/I
D-8000 München 22(DE)

(54) **Cytotoxic substances from the marine cyanophyte hormothamnion enteromorphoides grunow.**

(57) Several new, biologically active substances have been isolated from *Hormothamnion enteromorphoides*, a somewhat rare cyanophyte from Puerto Rico.

Hormothamnion, as lipophilic metabolite from this alga, has the styrylchromone structure:

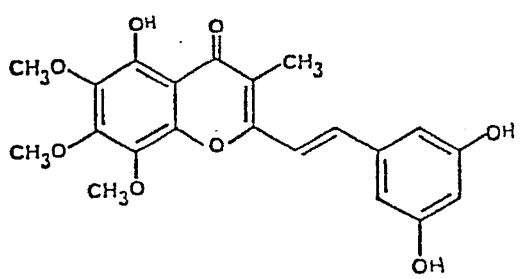

Hormothamnion and, to a lesser extent, one of its homologs, are potent cytotoxins to some types of cancer cells and appear to exert this cytotoxic action by a selective inhibition of RNA synthesis.

A peptide compound of about 11 to 12 amino acids has antimocrobial activity, particularly against *Candida albicans*. The peptide, which includes phenylalanine, serine and other aliphatic amino acids, has blocked carboxyl and amino termini.

EP 0 237 166 A2

# CYTOTOXIC SUBSTANCES FROM THE MARINE CYANOPHYTE *HORMOTHAMNION ENTEROMORPHOIDES* GRUNOW

## SUMMARY OF THE INVENTION

The present invention relates to biologically active substances obtainable from Cyanophyta, and more particularly to the chromone metabolites and peptides thereof.

The search for new structure types from marine organisms with potential drug activity has intensified in recent years, and has recently centered on several specific taxonomic groups, one of which is the Cyanophyta.

As described in the following publications, blue green algae have recently received considerable attention by academic researchers, the National Cancer Institute, and industry as a new source of novel bioactive natural products: Carter et al, *J. Org. Chem.* *49*:236-244 (1984); Moore et al., *J. Am. Chem. Soc.*, *106*:6456-6457 (1984); Barchi et al., *J. Am. Chem. Soc.*, *106*:8193-8197 (1984); Cragg et al., *Am. Soc. Pharmacog. Proceedings*, abs. no. 185 (1985); 1984 Prospectus, Sea Pharm, Inc., Ft. Pierce, Florida. Carter et al. have reported an abundance of unique secondary metabolites from the principal mat-forming marine cyanophyte, *Microcoleus lyngbyaceus* (syn *Lyngbya majuscula*).

Previously, styrylchromone structures have not been found in natural products although the carbon skeleton is known from synthetic studies as reported by G. P. Ellis in "Alkylchromones" in

Heterocyclic Compounds: Chromenes, Chromanones and Chromones, (Ellis, G. P., ed., John Wiley and Sons, New York, 1977, pp. 581-631).

It has now been found that Hormothamnion enteromorphoides, a somewhat rare cyanophyte from Puerto Rico, yields certain biologically active compounds.

A new lipophilic metabolite, named hormothamnion, is found to have a styrylchromone structure, which is without precedent among known natural products. Hormothamnion is a potent cytotoxin to some types of cancer cells ($IC_{50}$ = 0.2 ng/ml to the HL 60 cell line in tissue culture). It, and to a lesser extent, one homologous compound appear to exert this cytotoxic action by a selective inhibition of RNA synthesis. Hormothamnion represents the first of a new class of cytotoxins which are readily synthesizable.

Additionally, a peptide substance, having antimicrobial properties, has been isolated.

## DESCRIPTION OF THE DRAWING

In the drawing:

Fig. 1 is a perspective drawing of an x-ray model of hormothamnion triacetate.

## DETAILED DESCRIPTION

The marine cyanophycean alga Hormothamnion enteromorphoides Grunow (Nostocaceae) is one of only a few blue greens which form dense mats in shallow tropical marine environments. H. enteromorphoides as described herein was collected from the exposed north coast of Puerto Rico.

### A. Chromones.

At least two styrylchromone compounds from H. enteromorphoides were found to have antineoplastic drug activity.

### Example 1

A $CHCl_3$/MeOH extract of fresh H. enteromorphoides demonstrated slight gram positive antimicrobial activity and showed the presence of a distinctive orange-charring ($H_2SO_4$) yellow

pigment by thin layer chromatography. The readily isolable pigment band contained a subtle mixture of several compounds, the major of which was isolated by careful reverse phased HPLC and its novel styrylchromone structure (Fig. 1) solved by x-ray crystallographic means. Trivially named hormothamnion, this metabolite is a potent cytotoxin to several human cancerous cell lines and appears to operate via inhibition of RNA synthesis (Table I).

Table I. Inhibition of Cell Growth and Macromolecule Biosynthesis in Several Cancerous Human Cell Lines by Hormothamnione (1)

| Cell-line | $ID_{50}$ (ng/ml)[a] Cell Growth | $ID_{50}$ (ng/ml)[bc] DNA | $ID_{50}$ (ng/ml)[bd] RNA | $ID_{50}$ (ng/ml)[be] Protein |
|---|---|---|---|---|
| P-388 | 4.6 | --- | --- | --- |
| HL-60 | 0.1 | 300 | 1.0 | > 500 |
| KB | --- | 530 | 140 | > 500 |

[a] $ID_{50}$ is the drug concentration required to inhibit cell growth to 50% of control levels.

[b] The percentages of macromolecule biosynthesis inhibition were calculated from the specific activities of incorporated precursors (c-e) into $10^6$ cells in drug treated as versus control cells. $ID_{50}$ values were obtained by plotting the logarithmic drug concentrations against the percentage inhibition of macromolecule biosynthesis.

[c] Exponentially growing cells pulse labeled with 1 µCi of $^3$H-thymidine (16.6 Ci/mmole) per mL of cell suspension.

[d] Exponentially growing cells pulse labeled with 2 µCi [5-$^3$H]-uridine (28 Ci/mmole) per mL of cell suspension.

[e] Exponentially growing cells pulse labeled with 1 µCi of $^3$H-L-leucine (130 Ci/mmole) per mL of cell suspension.

Senescent yellow-colored H. enteromorphoides was found in abundance on an exposed shallow reef (1-3m) at Playa de Vega Baja, Puerto Rico several times during the months of July and August in 1984 and 1985. The frozen or alcohol preserved seaweed was extracted in standard fashion ($CHCl_3$/MeOH 2:1) and the yellow pigment band containing hormothamnion was isolated by vacuum chromatography over TLC grade silica gel. Preparative thick layer chromatography of this yellow band simplified the mixture to two components of 75:25 ratio. Small quantities of hormothamnion were obtained from this mixture employing painstaking reverse phased HPLC conditions using two Waters 3.9 mm x 25 cm µ-Bondapak C-18 columns in series, 40 percent $H_2O$/MeOH as mobil phase, 1.5 ml min., 35 min. retention time, 0.5 mg per injection. Hormothamnion was easily reformed (as determined by TLC, MS, NMR) via mild base treatment ($K_2KO_3$ in MeOH) of the derivative. The relative ease in purification of the derivatives and its more favorable solubility properties were factors in deciding to work largely the derivative rather than the natural product. Formulas of the natural product, hormothamnion (1) and its derivative (2) are as follows:

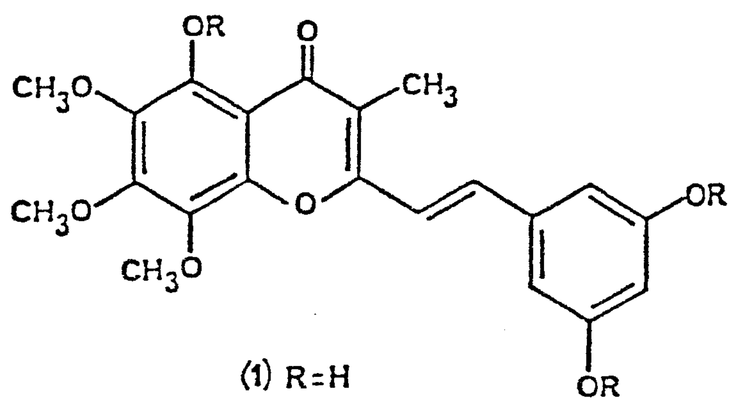

(1) R=H

(2) R=$\underset{\overset{\|}{O}}{C}CH_3$

Hormothamnion was a yellow solid (m.p. 270°C dec) which analyzed for $C_{21}H_{20}O_8$ by high resolution election impact mass spectrometry ($M^+$ M/Z 400.1163, 46 percent) which upon derivatization increased in mass to $C_{27}H_{26}O_{11}$ ($M^+$ = base peak at m/Z 526 by low resolution CIMS in the negative ion mode) and thus, the derivative was the triacetate derivative of hormothamnion. The natural product had typical chromone (Ellis, G. P., "Chromone and

Its Benzo Derivatives" in <u>Heterocyclic Compounds: Chromenes, Chromanones and Chromones</u>, G. P. Ellis, ed., New York, 1977; pp. 557-580). UV absorptions at 295 and 353 nm (MeOH; ε=6,200; 8,900 respectively) and IR absorptions (KBr) broadly centered at 3400 $cm^{-1}$ (phenol) and at 1640 $cm^{-1}$ (pyrone carbonyl). The $^1H$ NMR (80 MHz, $CDCl_3$) of the derivative confirmed its triacetate nature [δ 2.34 (6H,s) and 2.50 (3H,s)].

Of the remaining seventeen hydrogens in the $^1H$ NMR spectrum of the derivative, twelve were present as methyl singlets, three at shifts consistent with aromatic methoxyls [δ 3.89 (3H,s), 4.06 (3H,s) and 4.11 (3H,s)] and one consistent with a deshielded olefinic methyl group [δ 2.17 (3H,s)]. The other 5 protons were separated into two deshielded spin systems, one consistent with a trans disubstituted olefin [δ 7.08 (1H,d,J=16) and δ 7.58 (1H,d,J=16)] and the other interpreted as forming the proton component of a symmetrical 1,3,5 trisubstituted aromatic ring [δ 6.94 (1H,t,J=1.9) and 7.23 (2H,d,J=1.9)]. The nature of the ring system and placement of these proton, methyl, methoxy and acetoxy substituents was not readily determinable, and more definitive NMR experiments were precluded due to limited resources of compound. Hence, an x-ray experiment was performed to unambiguously assign the structure of the crystalline derivative (mp 198-201°C).

Preliminary x-ray photographs of the triacetate derivative displayed monoclinic symmetry and accurate lattice constants of a=18.472(5), b=5.769(3), c=24.096(6) Å, and $\beta$=93.40(2)° were determined from a least squares analysis of fifteen moderate, diffractometer measured 2θ-values. The systematic extinctions and crystal density indicated space group $P2_{1/a}$ with one molecule of composition $C_{27}H_{26}O_{11}$ forming the asymmetric unit (Z=4). All diffraction maxima with 2θ < or = 114° were measured on a computer controlled four-circle diffractometer with graphite monochromated Cu Kα radiation (1.54178 Å) and variable speed, 1° ω-scans. Of the 3459 reflections surveyed, only 1211 (35 percent) were judged observed ($F_o$ > or = $3\sigma(F_o)$) after correcton for Lorentz, polarization and background effects. The centrosymmetric structure was solved easily. Hydrogen atoms were located on a ΔF-synthesis after partial refinement of the nonhydrogen atoms. Block diagonal

lease-squares refinements with anisotropic nonhydrogen atoms and isotropic hydrogens have converged to a standard crystallographic residual of 0.081 for the observed reflections.

A computer generated drawing of the final x-ray model of hormothamnion triacetate appears in Fig. 1. The chromone fragment (atoms O1 to C8a) is planar, and the substitutents on the fully substituted aromatic ring are all rotated out of the plane. The relevant torsional angles vary from 50° for C8-C7-O31-C21 to 87° for C7-C8-O32-C22. The acetate substituents on the 1,3,5-substituted ring are also rotated by roughly 77°.

As indicated in Table I, hormothamnion was found to be a potent cytotoxic agent to P388 lymphocytic leukemia and HL-60 human promyelocytic leukemia cell lines. Macromolecule biosynthesis in the presence of hormothamnion was measured via radioactive precursor incorporation studies using HL-60 and KB cell lines. A major mode of cytotoxic action of hormothamnion appears to be by inhibition of RNA synthesis.

Hormothamnion showed minimal gram positive antimicrobial activity when tested by the paper disc-agar plate method, using 100 µg and 25 µg of hormothamnion per 6 mm paper disc, tested against Staphylococcus aureus (-), Bacillus subtilus (10 mm zone at 100 µg, 8 mm zone at 25 µg), Escherichia coli (-) and Candida albicans (-).

Example 2

A closely related styrylchromone compound was isolated and identified from the of Example 1 using the same procedures:

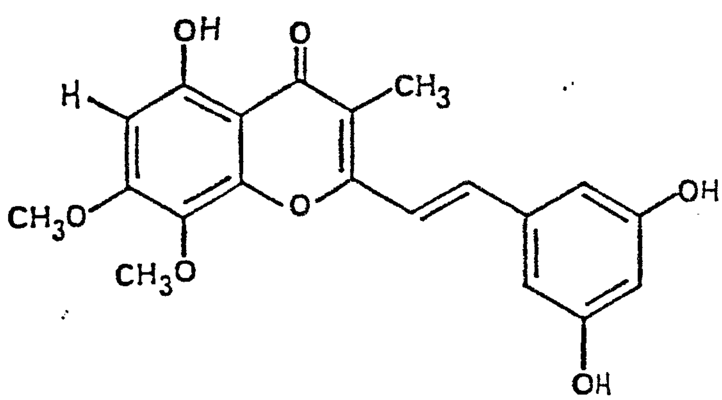

(3)

When tested, the compound of formula (3) also inhibited the growth and macromolecule biosynthesis of several human cell lines. But, the effect was less than observed with hormothamnion.

Styrylchromone substances can be administered to animal subjects in any convenient dosage form. Salts or esters of the compounds may be preferred for use in medications.

Multiple collections of Hormothamnion enteromorphoides from different seasons has shown that hormothamnion compounds are present only in yellow senescent growths of the alga found during the late summer. At other times of the year, the alga appears dark green and produces different natural products.

## B. Peptides

One of the natural products found from Hormothamnion enteromorphoides harvested during the spring, particularly in March, is a new peptide with apparent antimicrobial activity.

## Example 3

H. enteromorphoides, collected in March of 1985, was extracted as described in Example 1 ($CHCL_3$/MeOH 2:1). The extract was found to inhibit growth and reproduction of certain microorganisms, most notably Candida albicans. Accordingly, various substances were isolated from the extract by thin layer chromatography analysis to identify the active compound. The active compound was found to be a peptide consisting of about 11 or 12 amino acids, including phenylalanine, serine, and several other aliphatic amino acids. The peptide is probably of a cyclic nature and appears to have blocked carboxyl and amino termini.

Peptides of the type described herein can be used in the manner of other known organic antimicrobial agents.

While I have shown and described the preferred embodiments of my invention, it will be apparent to those skilled in the art that changes and modifications may be made without departing from my invention in its broader aspects. I therefore intend the appended claims to cover all such claims and modifications as follow the true spirit and scope of my invention.

The features disclosed in the foregoing description, in the following claims and/or in the accompanying drawings may, both separately and in any combination thereof, be material for realising the invention in diverse forms thereof.

CLAIMS

1. A compound of the formula:

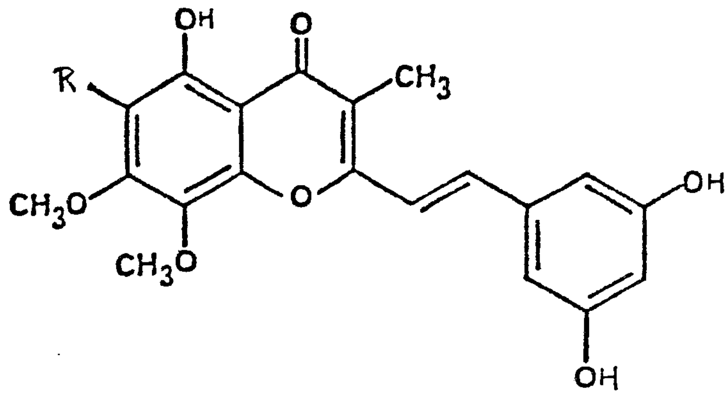

wherein R is -H or $-OCH_3$.

2. A method of treating a human or other animal subject to inhibit the proliferation of malignant cells, the method comprising administering to the subject an effective amount of a chromone.

3. The method of claim 2 wherein the chromone is a compound of the formula:

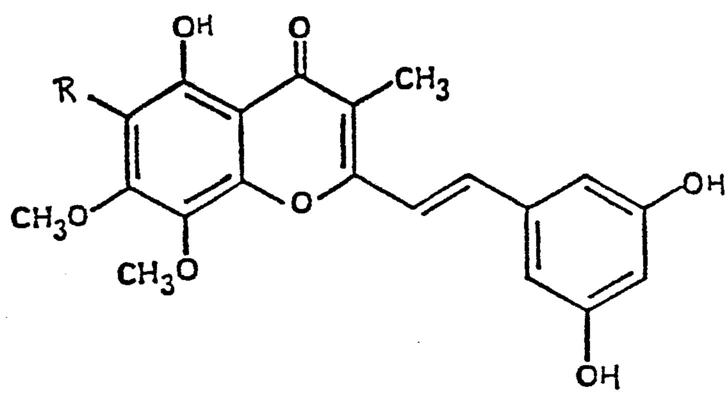

wherein R is -H or $-OCH_3$.

4. A composition of matter comprising a pharmaceutically inert carrier and a chromone compound in an amount sufficient to inhibit the proliferation of malignant cells.

5. The composition of matter of claim 4 wherein the chromone is a compound of the formula:

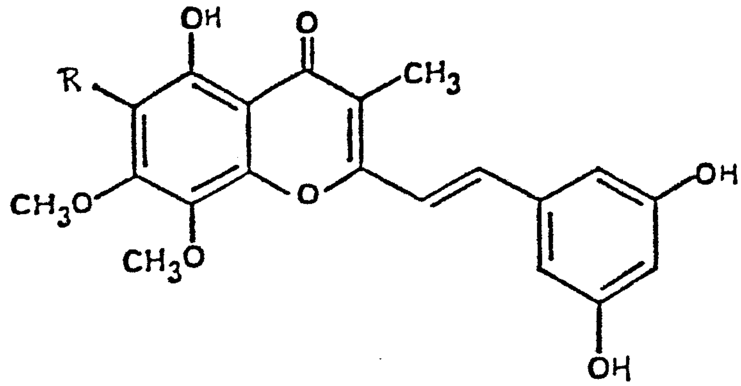

wherein R is -H or $-OCH_3$.

6. A peptide characterized in that it:

is extractable by a mixture of chloroform and methanol from cells of Hormothamnion enteromorphoides that are harvested from the waters of Puerto Rico during the month of March; and

inhibits Candida albicans.

7. The peptide of claim 6 further characterized in that it:

consists of about 12-13 amino acids, including phenylalanine, serine, and multiple additional aliphatic amino acids;

has a blocked carboxyl terminus; and

has a blocked amino terminus.

8. The peptide of claim 7 further characterized in that it is a cyclic peptide.

9. A composition of matter comprising a pharmaceutically inert carrier and an amount of the peptide of claim 6 sufficient to inhibit the propagation of a microorganism.

10. An antimicrobial peptide substance obtained by the steps of:

harvesting cells of Hormothamnion enteromorphoides from the waters of Puerto Rico during the spring;

contacting the cells with a mixture of chloroform and methanol to form an extract solution; and

separating the chloroform and methanol from the extract solution, whereby a crude extract, containing the peptide, is obtained.

11. A process for inhibiting microbial growth and reproduction, the process comprising administering, in an amount to inhibit microbial growth and reproduction, a peptide characterized in that it:

is extractable by a mixture of chloroform and methanol from cells of Hormothamnion enteromorphoides that are harvested from the waters of Puerto Rico during the month of March; and

inhibits Candida albicans.

0237166

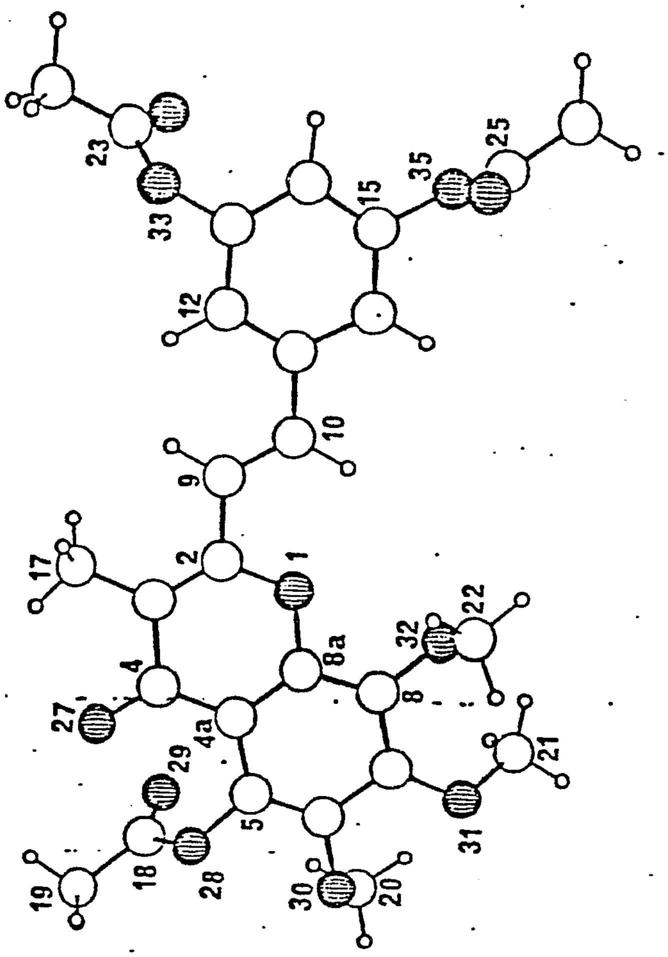

FIG. 1